# EUROPÄISCHE PATENTSCHRIFT

(11) **EP 1 473 083 B1**
(45) Veröffentlichungstag und Bekanntmachung des Hinweises auf die Patenterteilung: **23.05.2012**
(21) Anmeldenummer: 04007058.3
(22) Anmeldetag: 24.03.2004
(51) Int. Cl.: B01J 37/08, B01J 37/02, B01J 27/22, B01J 23/30, B01J 23/36, B01J 23/28, C07C 6/04, C07C 11/02, C07C 11/06, C07C 11/107

(54) **Verfahren zur Herstellung von aktivierten Methathesekatalysatoren**
Process for preparing activated metathesis catalysts
Procédé de préparation de catalyseurs de metathèse activés

(30) Priorität: 30.04.2003 DE 10319439
(43) Veröffentlichungstag der Anmeldung: 03.11.2004
(73) Patentinhaber: BASF SE, 67056 Ludwigshafen (DE)
(72) Erfinder: Schubert, Markus, Dr., 67063 Ludwigshafen (DE); Hesse, Michael, Dr., 67549 Worms (DE); Stephan, Jürgen, Dr., 68163 Mannheim (DE); Böhm, Volker, Dr., 67227 Frankenthal (DE); Brodhagen, Andreas, Dr., 67125 Dannstadt-Schauernheim (DE); Poplow, Frank, Dr., 67065 Ludwigshafen (DE); Sinner-Lang, Martina, 67071 Ludwigshafen (DE); Diehlmann, Uwe, 67454 Hassloch (DE); Cox, Gerhard, Dr., 67098 Bad Dürkheim (DE); Pfeifer, Jochen, 76831 Ilbesheim (DE)

(56) Entgegenhaltungen:
- FR-A- 1 370 025
- US-A- 3 546 314
- US-A- 3 952 070
- US-A- 4 709 115
- US-A- 5 196 389

## Beschreibung

Die vorliegende Erfindung betrifft Verfahren zur Herstellung von Trägerkatalysatoren (Katalysator K), umfassend einen Träger (Träger T) und eine Aktivkomponente (Aktivator A), wobei man
a) einen Katalysator-Precursor herstellt, indem man auf einen Träger ausgewählt aus der Gruppe bestehend aus Al₂O₃, Alumosilicate, Ga₂O₃, SiO₂, GeO₂, TiO₂, ZrO₂, SnO₂ und Mischungen der vorgenannten Verbindungen, einen für die Katalyse von Metathesereaktionen üblichen Aktivator (Aktivator-Precursor), der Verbindungen umfasst, die Elemente aus der V., VI. und VII. Nebengruppe enthalten, aufbringt (Schritt a)
b) den gemäß Schritt (a) hergestellten Katalysator-Precursor mit einem C₃- bis C₁₂-Olefin bei einer Temperatur zwischen -20 und 550°C in Kontakt bringt (Schritt b) und
c) den gemäß Schritt (b) hergestellten Katalysator-Precursor in einer Inertgasatmosphäre bei einer Temperatur von 410 bis 850°C tempert (Schritt c).

Die Metathese von ungesättigten Verbindungen ist eine seit langem etablierte Methode, um C-C-Verbindungen aufzubrechen und neu zu knüpfen (z. B. Mol, J. C., Chapt. 4.12.2 "Alkene Metathesis" in "Handbook of Heterogeneous Catalysis", Eds. Ertl, G., Knözinger, H., Weitkamp, J., VCH, Weinheim 1997; Weissermehl, K., Arpe, H.-J., Chapt. 3.4 "Olefin-Metathese" in "Industrielle Organische Chemie", 4. Auf)., VCH, Weinheim 1994).

Für eine heterogen katalysierte Metathese wurden verschiedene Typen von Katalysatoren beschrieben. Für den Temperaturbereich bis zu ca. 120°C ist die Verwendung geträgerter Re₂O₇- oder Re(CO)₁₀-Katalysatoren üblich (Mol, J. C., Chapt. 4.12.2 "Alkene Metathesis" in "Handbook of Heterogeneous Catalysis", Eds. Ertl, G., Knözinger, H., Weitkamp, J., VCH, Weinheim 1997). Rhenium ist jedoch ein seltenes und relativ teures Element, so daß der Einsatz eines solchen Katalysators oftmals unwirtschaftlich ist. Bei etwas höheren Temperaturen von bis zu 400°C können der Literatur zufolge Katalysatoren auf Basis MoO₃, CoO-MoO₃, MoS₂, Mo(CO)₆ oder diverse geträgerte Mo-Komplexe angewandt werden, bei noch höheren Temperaturen von bis zu 540°C auch Systeme auf Basis WO₃, WS₂, W(CO)₆ oder geträgerter W-Komplexe (Mol, J. C., Chapt. 4.12.2 "Alkene Metathesis" in "Handbook of Heterogeneous Catalysis", Eds. Ertl, G., Knözinger, H., Weitkamp, J., VCH, Weinheim 1997; Weissermehl, K., Arpe, H.-J., Chapt. 3.4 "Olefin-Metathese" in "Industrielle Organische Chemie", 4. Aufl., VCH, Weinheim 1994; Heckelsberg, L. F., Banks, R. L., Bailey, G. C., Ind. Eng. Chem. Prod. Res. Develop. 8 (1969), 259 - 261). Diese sind zwar sehr preiswert, weisen jedoch generell eine deutlich geringere Aktivität auf und zeigen zudem niedrigere Selektivitäten. Die reduzierten Selektivitäten sind auf die Nebenreaktion der Doppelbindungsisomerisierung zurückzuführen, die an den stark sauren Molybdän- und Wolframverbindungen bei höheren Reaktionstemperaturen parallel zur Metathese abläuft, was zur Bildung unerwünschter Produkte führt.

Um die Nebenreaktion der Doppelbindungsisomerisierung zu unterdrücken, wird in US 3,586,731 der Zusatz von Alkali- oder Erdalkalisalzen zu Silca-geträgerten Oxiden, Sulfiden oder Hexacarbonylen von Wolfram, Molybdän oder Rhenium beschrieben. Dies kann allerdings zu einem beträchtlichen Absinken der Katalysatoraktivität führen.

In US 4,024,201 wird vorgeschlagen, dem Feed für einen geträgerten WO₃-Katalysator halogenhaltige Verbindungen oder Amine zuzusetzen. Solche polaren Verbindungen sind jedoch auch gleichzeitig als Katalysatorgifte in der Metathese bekannt, daher ist auch hier eine stark erniedrigte Aktivität zu erwarten.

US-A-3,952,070 offenbart ein Verfahren zur Olefin-Metathese, in dem ein Katalysator eingesetzt wird, welcher hergestellt wird, indem ein Trägermaterial ausgewählt aus Silika, Alumina, Thoria, Zirkonia, Titania, Aluminiumphosphat, Zirkoniumphosphat, Calciumphosphat, Magnesiumphosphat oder Titanphosphat mit einer Lösung einer geeigneten Wolfram-Verbindung imprägniert wird. Dieses Material wird dann mit Luft oder Sauerstoff bei 500 bis 750°C calciniert, um das Wolframsalz in Wolframoxid umzuwandeln, Der so erhaltene calcinierte Katalysator mit Ethen oder einem Buten bei einer Temperatur von 450°C bis 750°C und einem Druck von 0 bis ungefähr 500 psig behandelt, so dass der erhaltene Katalysator sofort ohne Induktionszeit eine hohe Aktivität bei der Metathese aufweist.

US-A-3,546,314 offenbart ein Verfahren zur Umwandlung von ungesättigten Kohlenwasserstoffen in andere ungesättigte Kohlenwasserstoffe in Gegenwart eines geeigneten Katalysators, beispielsweise Wolframoxid auf Silika. Dieser Wolframoxid-Silika-Katalysator wird hergestellt, indem Silika mit einer entsprechenden Wolfram-Verbindung imprägniert wird, und diese in das entsprechende Oxid umgewandelt wird.

US-A-4,709,115 offenbart ein Verfahren zur Disproportionierung von Alkinen, in dem Katalysatoren eingesetzt werden können, welche katalytisch aktive Metalle wie Rhenium, Molybdän oder Wolfram und gegebenenfalls einem Promoter enthalten. Als Trägermaterial dieser Katalysatoren ist Alumina oder Silika-Alumina geeignet. Die Katalysatoren werden erhalten, indem geeignete lösliche Verbindungen auf das Trägermaterial aufgebracht werden, durch Calcinierung diese Verbindungen in die entsprechenden Oxide umgewandelt werden, und durch Erhitzen in einem Strom von inerten Gasen aktiviert werden.

US-A-5,196,389 offenbart ein katalytisch aktives System zur Verwendung in Abgasanlagen zur katalytischen Zersetzung von Abgasen. Dazu wird ein metallisches Carbid als Katalysator eingesetzt, welches erhalten wird durch Beschichten eines entsprechenden Trägermaterials mit einer Suspension der reduzierbaren Verbindung des Metalls in einer Lösung einer organischen Verbindung, und anschließender Verkohlung dieser Verbindung, Reduktion der metallischen Verbindung und Carburetisierung. Dazu wird ein poröses Trägermaterial mit einer Suspension der Vorläufer-Verbindungen des katalytisch aktiven Metalls und einer organischen Verbindung beschichtet, das Lösungsmittel wird durch Erhitzen in einem Ofen entfernt, so dass ein Katalysator-Vorläufer erhalten wird, in dem Metalloxide in einer organischen Matrix vorliegen. Durch Verkohlung der organischen Matrix wird ein Katalysator erhalten, in dem metallische Oxide in einer Matrix von Kohlestoff vorliegen. Abschließend werden die Metalloxide zu den Metallen reduziert und carburetiert, so dass auf einem Siliziumcarbid-Grundgerüst eine Schicht von metallischen Carbiden vorliegt.

Erfindungsgemäße Aufgabe war deshalb die Herstellung eines Katalysators mit erhöhter Metatheseaktivität und Selektivität.

Demgemäß wurden die eingangs definierten Verfahren und Katalysatoren gefunden.

Bei den Katalysator-Precursoren, die gemäß Schritt a) hergestellt werden und anschließend gemäß den Schritten b) und c) zu den erfindungsgemäßen Katalysatoren K weiter umgesetzt werden, handelt es sich um die üblicherweise in Metathesereaktionen eingesetzten Trägerkatalysatoren. Solche Katalysatoren sind z.B. beschrieben in "Handbook of Heterogeneous Catalysis", Herausgegeben von G. Ertl, H. Knözinger und J. Weitkamp, Volume 5, VCH Verlagsgesellschaft mbH, Weinheim, Kapitel 4.12.2, Alkene Metathesis, Seiten 2387 bis 2399.

Als Träger (Träger T) für die Herstellung der Katalysator-Precursoren werden solche ausgewählt aus der Gruppe bestehend aus Al₂O₃, Alumosilicate, Ga₂O₃, SiO₂, GeO₂, TiO₂, ZrO₂, SnO₂ und Mischungen der vorgenannten Verbindungen eingesetzt. Geeignete Träger weisen typischerweise ein spezifische Oberfläche von 10 - 500 m²/g, bevorzugt 100 - 400 m²/g auf. Das bevorzugte Porenvolumen (bestimmt mittels Quecksilberporsosimetrie) liegt zwischen 0.3 und 1,3 ml/g. Die bevorzugte Wasseraufnahme beträgt 0,5 bis 1,5 ml/g. Bei dem Träger handelt es sich üblicherweise um Formkörper wie Kugeln, Splitt, Stränge oder Tabletten. Der Träger kann gegebenenfalls mit Säuren vorbehandelt sein.

Die in Schritt a) auf den Träger (T) aufgebrachten Aktivkomponenten (Aktivator-Precursoren) umfassen die üblicherweise verwendeten Verbindungen sowie Mischungen hiervon. Dabei handelt es sich um Verbindungen der Metalle der V., VI. oder VII. Nebengruppe, insbesondere Verbindungen des Rheniums, Wolframs oder Molybdäns. In Betracht kommen dabei die Sulfide, Oxide, Nitride, Carbide, Oxycarbide, Carbonyle, organische Komplexe, Halogenide, Säuren, Polysäuren, Heteropolysäuren und Salze der Säuren, Polysäuren und Heteropolysäuren. Solche Salze sind bevorzugt Alkali- oder Ammoniumsalze. Als organische Komplexe werden in diesem Zusammenhang beispielsweise Dialkylkomplexe, Acylverbindungen, Acetylacteonate, oder Allylkomplexe verstanden. Besonders bevorzugt ist Molybdänoxid und Wolframoxid. Die Bezeichung Salze schließt auch substöchiometrische Bronzen mit ein. Die Bezeichnung Oxid erstreckt sich hierbei nicht nur auf die stöchiometrischen Verbindungen wie MoO₃, WO₃, MoO₂ und WO₂, sondern schließt auch substöchiometrische Phasen des Typs MO₃₋₂ mit ein. Als Träger für Wolfram- oder Molybdänverbindungen kommt ganz besonders bevorzugt SiO₂ in Betracht.

Im Allgemeinen geht man bei der Herstellung der als Katalysator-Precursoren dienenden üblichen Katalysatoren mit Oxiden als Aktivator-Precursoren gemäß Schritt a) so vor, dass man den Träger T mit einer Lösung der vorgenannten Verbindungen imprägniert. Im Fall von Wolframoxid dient hierzu z.B. eine Lösung von Ammoniummetawolframat, Wolframsäure oder Wolframpentachlorid. Die imprägnierten Träger werden anschließend meistens mehrere Stunden bei Temperaturen von 100 bis 200°C an der Luft getrocknet. Danach folgt üblicherweise ein Calcinierungsschritt. Hierzu werden die imprägnierten und getrockneten Träger üblicherweise in einer Sauerstoffhaltigen Gasatmosphäre, z.B. an der Luft, auf Temperaturen von 400 bis 850°C über einen Zeitraum von etwa einer halben Stunde bis 5 Stunden erhitzt. Die derart hergestellten Katalysator-Precursoren können auch noch mit Temperschritten in Inertgas, beispielsweise N₂, CO₂ oder Edelgase, vorbehandelt werden oder in reduierenden Gasmischungen, die beispielsweis Wasserstoff, CO, Ammoniak oder Hydrazin enthalten, teilweise reduziert werden.

Zur Herstellung der zu den Katalysator-Precursoren zählenden üblichen Trägerkatalysatoren mit Carbiden oder Oxicarbiden als Aktivator-Precursoren geht man üblicherweise von den nach der obigen Methode hergestellten Katalysator-Precursoren aus, die als Aktivkomponenten Oxide tragen. Zur Carbidisierung werden die entsprechenden metalloxidtragenden Katalysator-Precursoren in einem kohlenwasserstoffhaltigen Strom, z.B. einem Methanstrom, in Gegenwart von Wasserstoff im Allgemeinen mehrere Stunden bei Temperaturen von 550 bis 800°C erhitzt. Zur Herstellung der Wolframcarbide werden typischerweise um etwa 50 - 200°C höhere Temperaturen als zur Herstellung der Molybdäncarbide benötigt. Die Eigenschaften der Carbide werden auch durch das H₂/CH₄-Verhältnis beeinflusst, ein typischer Wert liegt hier bei 80/20. Die entsprechenden Carbidisierungsmethoden sind bekannt und z.B. in Oyama, S. T., Catal. Today, 15 (1992), 179 beschrieben.

Nach der Carbidisierung müssen diese Katalysator-Precursoren wegen ihrer Luftempfindlichkeit unter Inertgasatmosphäre gelagert werden, oder mit verdünntem Sauerstoff passiviert und dann im Synthesereaktor reaktiviert werden. Eine weitere Möglichkeit besteht in dem Ausbau der frisch hergestellten Carbide unter einer Flüssigkeit, die die Carbidoberfläche vor Luftsauerstoff weitgehend schützt.

Weiterhin eignen sich folgende Verfahren zur Herstellung von Katalysator-Precursoren, die als Aktivator-Precursoren Carbide tragen:

Lee et al. beschreiben in J. Catal. 128, 126 (1991) die Herstellung von Al₂O₃-geträgerten Molybdäncarbiden durch (i) Reduktion gefolgt von Carbidisierung, (ii) direkte Carbidisierung in CH₄/H₂ oder (iii) Nitridierung mit NH₃ gefolgt von der Carbidisierung.

In Volpe, Boudart, J. Solid State Chem. 59, 332 (1985) und Volpe, Boudart, J. Solid State Chem. 59, 348 (1985) wird die Nitridierung/Carbidisierung von MoO₃ und WO₃ näher beschrieben.

Die Reduktion von MoO₃ auf Kohle-Trägern durch Wasserstoff, die mit einer Carbidisierung durch den Kohleträger ab Temperaturen von 530°C gekoppelt ist, wird etwa in Liang et al., Chem. Mater. 2002,14, 3148 beschrieben.

Was die Oxicarbide betrifft, die als Aktivator-Precursoren Oxicarbide in Betracht kommen, so sind diese z.B.: in Pham-Huu et al., Appl. Catal. A 132 (1995), 77 beschrieben. Die Herstellung kann entweder aus den Oxiden durch nur teilweise Carbidisierung erfolgen. Die Oxicarbide bilden sich unter geeigneten Bedingungen auch während der Reaktion, wenn vom Oxid ausgegangen wird und dann ein Kohlenwasserstoff/H₂-Gemisch über den Katalysator bei erhöhten Temperaturen geleitet wird (etwa: H₂/n-Hexan = 150, T = 350°C).

Die Oxicarbide können auch hergestellt werden durch Sauerstoffbehandlung von Carbiden. In Ledoux et al., New Frontiers in Catalysis, 1993, S. 955, Guczi, L. et al (Editors), Elsevier Science Publishers B.V., wird das Carbid bei 350°C erst mit Luft und dann bei derselben Temperatur mit Wasserstoff behandelt.

Gemäß Schritt b) werden die so hergestellten Katalysator-Precursoren mit einem C₃-bis C₁₂-Olefin, ganz besonders Butene und Oktene, wie z.B. 1-Buten und n-Okten-1, in Kontakt gebracht.

Bei der Behandlung des Katalysator-Precursors mit einem C₃- bis C₁₂-Olefin kann diese sowohl flüssig als auch gasförmig vorliegen. Die Behandlungsdauer ist unkritisch und beträgt üblicherweise 1 min bis 24 h, bevorzugt 5 min bis 4 h. Die Temperatur während der Behandlung beträgt -20 bis 550°C, sie ist jedoch unkritisch. Letzteres gilt im Allgemeinen auch für den Druck, er beträgt im Allgemeinen 0,5 bis 40 bar.

Der mit Kohlenwasserstoff vorbehandelte Katalysator-Precursor wird anschließend gemäß Schritt c) in einer Inertgasatmosphäre auf eine Temperatur von 410 bis 850, bevorzugt 500 bis 850°C erhitzt. Geeignete Inertgase sind vor allem Stickstoff, CO₂ sowie die Edelgase. Die Behandlung gemäß Schritt c) erfolgt üblicherweise über einen Zeitraum von 5 min bis 100 h, bevorzugt 30 min bis 24 h, wobei der Druck hier wiederum ebenfalls unkritisch ist und üblicherweise 0,5 bis 40 bar beträgt.

Die erfindungsgemäßen Katalysatoren (K) können zusätzlich auch Promotoren enthalten. Hierbei handelt es im Allgemeinen um Cobalt-, Alkali-oder Erdalkalimetallverbindungen. Sie werden im Allgemeinen auf den Katalysator aufgebracht, indem man den Tränklösungen zur Herstellung der Katalysator-Precursoren entsprechende Salze, z.B. Nitrate oder Hydroxide beifügt oder die Katalysatoren mit einer entsprechenden Tränklösung nachträglich dotiert und zur Fixierung nochmals calciniert.

Der Anteil des Aktivators (A) am Katalysator (K) beträgt üblicherweise 0,1 bis 30 Gew.-%.

Besonders bevorzugt besteht der Katalysator (K) aus WO₃ in tetragonaler Form als Aktivator (A) und SiO₂ als Träger (T).

Die erfindungsgemäßen Katalysatoren eignen sich insbesondere für die Metathese von ungesättigten Verbindungen, wie Alkenen oder Alkinen. Solche Verfahren sind allgemein bekannt und z.B. in "Industrielle Organische Chemie", Klaus Weissermel, Hans-Jürgen Erpel, 5. Auflage, Verlag Wiley, VCH, 1998, Kapitel 3.4 und Handbook of Heterogeneous Catalysis", Herausgegeben von G. Ertl, H. Knözinger und J. Weitkamp, Volume 5, VCH Verlagsgesellschaft mbH, Weinheim, Kapitel 4.12.2, Alkene Metathesis, Seiten 2387 bis 2399, beschrieben. Sie können aber auch zur Metathese von ungesättigten Estern, Nitrilen, Ketonen, Aldehyden, Säuren oder Ethern eigesetzt werden, wie es beispielsweise in Xiaoding, X., Imhoff, P., von den Aardweg, C. N., and Mol, J. C., J. Chem. Soc., Chem. Comm. (1985), p. 273 beschrieben ist. Häufig wird bei der Umsetzung von substituierten Olefinen ein sogenannter Co-Katalysator, beispielsweise Zinn-, Blei- oder Aluminiumalkyle eingesetzt, um die Aktivität zusätzlich zu steigern.

Hierbei können die erfindungsgemäßen Katalysatoren (K) auf die gleiche Weise eingesetzt werden wie die bekannten Metathesekatalysatoren, die gemäß Schritt a) des erfindungsgemäßen Verfahrens hergestellt werden und als Katalysator-Precursoren der erfindungsgemäßen Katalysatoren K dienen.

Besonders günstig können die erfindungsgemäßen Katalysatoren in Metatheseverfahren zur Herstellung von Propen durch Metathese einer Mischung, die 2-Buten und Ethylen oder 1-Buten und 2-Butene enthält, sowie von 3-Hexen und Ethylen durch Metathese von 1-Buten eingesetzt werden. Entsprechende Verfahren sind im Detail in DE-A-19813720, EP-A-1134271, WO 02/083609, DE-A-10143160 beschrieben.

Die vorgenannten C₄-Ausgangsverbindungen werden üblicherweise in Form eines sogenannten Raffinat II bereitgestellt. Bei dem Raffinat II handelt es sich um C₄-Schnitte, die im Allgemeinen einen Gehalt an Butenen von 30 bis 100, bevorzugt 40 bis 98 Gew.-% aufweisen. Neben den Butenen können vor allem noch gesättigte C₄-Alkane vorhanden sein. Die Gewinnung solcher Raffinate II ist allgemein bekannt und z.B. in der EP-A-1134271 beschrieben.

Insbesondere kann 1-Buten eingesetzt werden, das durch Abdestillieren einer 1-Buten reichen Fraktion aus Raffinat II, gewonnen wird. Aus der verbleibenden, an 2-Butenen reichen Fraktion kann ebenfalls 1-Buten gewonnen werden, indem man die 2-Buten reiche Fraktion einer Isomerisierungsreaktion unterwirft und anschließend destillativ in eine 1-Buten und eine 2-Buten reiche Fraktion auftrennt. Dieses Verfahren ist in der DE-A-10311139 beschrieben.

Die erfindungsgemäßen rheniumhaltigen Katalysatoren eignen sich insbesondere für Reaktionen in flüssiger Phase, bei Temperaturen von 10 bis 150°C bei einem Druck von 5 bis 100 bar.

Die erfindungsgemäßen wolfram- bzw. molybdänhaltigen Katalysatoren werden im Allgemeinen bei Gasphasenreaktionen eingesetzt. Die Temperatur liegt hier im Allgemeinen bei 150 bis 500°C. Der Druck beträgt im Allgemeinen 5 - 50 bar.

Die vorliegende Erfindung betrifft bevorzugt das erfindungsgemäße Verfahren, wobei die Verbindungen der V., VI. und VII. Nebengruppe ausgewählt sind aus der Gruppe bestehend aus Sulfiden, Oxiden, Nitriden, Carbiden, Oxycarbiden, Carbonylen, organischen Komplexen, Halogeniden, Säuren, Polysäuren, Heteropolysäuren und Salzen der Säuren, Polysäuren und Heteropolysäuren.

Die vorliegende Erfindung betrifft weiter bevorzugt das erfindungsgemäße Verfahren, wobei der Aktivator-Precursor Verbindungen umfasst, die ausgewählt sind aus der Gruppe bestehend aus Rhenium- Wolfram- und Molybdänverbindungen.

Die vorliegende Erfindung betrifft weiter bevorzugt das erfindungsgemäße Verfahren, wobei der Aktivator-Precursor Verbindungen umfasst, die ausgewählt sind aus der Gruppe bestehend aus Molybdänoxiden, Wolframcarbiden und Wolframoxiden.

Die vorliegende Erfindung betrifft weiter bevorzugt das erfindungsgemäße Verfahren, wobei der Aktivator-Precursor Verbindungen umfasst, die ausgewählt sind aus der Gruppe bestehend aus Metallverbindungen der V., VI, und VII. Nebengruppe und einen Promotor, der ausgewählt ist aus der Gruppe bestehend aus Cobalt-, Alkali- und Erdalkalimetallverbindungen.

Die vorliegende Erfindung betrifft weiter bevorzugt das erfindungsgemäße Verfahren, wobei man den Schritt (a) ausführt, indem man den Träger (T) mit einer Lösung des Aktivator-Precursors imprägniert und anschließend trocknet und danach ggf. calciniert.

Die vorliegende Erfindung betrifft weiter bevorzugt das erfindungsgemäße Verfahren, wobei man in Schritt (c) ein Inertgas, ausgewählt aus der Gruppe bestehend aus Stickstoff, Kohlendioxid und Edelgas oder Mischungen hiervon einsetzt.

### Experimenteller Teil

### A. Herstellung der Katalysatoren

### A.1. Herstellung der Katalysator -Precursoren

### Beispiel 1: Herstellung von WO₃/SiO₂-Katalysatoren - Kats A - E

SiO₂-Träger wurden jeweils mit wäßriger, verdünnter Ammoniummetawolframatlösung auf Wasseraufnahme getränkt. Danach wurden die Stränge 16 h im Trockenschrank bei 120°C getrocknet. Abschließend wurde der Katalysator am Drehrohrofen unter den angegebenen Bedingungen behandelt und unter trockenem Stickstoff (20 Uh) abgekühlt Der Katalysator C1 wurde danach zusätzlich mit einer 0,5 M NaOH-Lösung getränkt und nochmals bei den genannten Bedingungen getrocknet und calciniert, wodurch der Na-Gehalt im fertigen Katalysator von 1100 auf 4500 ppm erhöht wurde (= Probe C2). Weitere Details über die Herstellungsbedingungen und die Katalysator-Precursoren selbst sind Tabelle 1 zu entnehmen

**Tabelle 1**

| Kat | WO₃-Gehalt [wt%] | Träger | Bedingungen i. Drehrohr |
|---|---|---|---|
| A | 12,1 | BASF D11-10, 1.5 mm Stränge (171 m²/g) | 1 h Luft (20 Uh), 593°C |
| B | 13,9 | BASF D11-10, 1.5 mm Stränge (171 m²/g) | 1 h Luft (20 Uh), 593°C |
| C1 | 12,5 | BASF D11-10, 1.5 mm Stränge (171 m²/g) | 1 h Luft (20 Uh), 593°C |
| D | 11,2 | Shell X970 CY, 3 mm Stränge (326 m²/g) | 1 h Luft (20 Uh), 593°C |
| E1 | 15,0 | Solvay Siligel BR 5155/1, 0.8 - 2 mm Kugeln (350 m²/g) | 1 h Luft (20 Uh), 593°C |
| E2 | 15,0 | Solvay Siligel BR 5155/1, 0.8 - 2 mm Kugeln (350 m²/g) | 1 h Luft (20 Uh), 593°C + 2.5 h N₂ (20 Uh), 850°C |
| F | 12,2 | BASF D11-10, 0.5-0.8 mm Splitt (171 m²/g) | 1 h Luft (20 Uh), 593°C |

### Beispiel 2: Herstellung des Wolframcarbid-Katalysators - Kat G

Zur Herstellung von Katalysator G wurde in einen von oben nach unten durchströmten Glasreaktor 70 ml des WO₃/SiO₂-Katalysators A eingefüllt. Der Glasreaktor wurde von außen durch einen elektrischen Ofen beheizt, die Katalysatorschüttung befand sich etwa in der Mitte der Heizzone auf einer Glasfritte. Nach Einbau des Katalysators und Verschließen des Reaktors wurde die Anlage zunächst mit Stickstoff gespült (30 min, 20 L/h). Anschließend wurde ein Gasstrom bestehend aus 3,9 Uh Methan und 15 L/h Wasserstoff über den Katalysator geleitet. Der Reaktor wurde dann innerhalb 180 min auf 750°C erhitzt und 6 h bei 750°C gehalten. Danach wurde innerhalb 1 h auf 500°C abgekühlt und diese Temperatur 2 h gehalten. Dann wurde abgekühlt und der Methan/Wasserstoffstrom durch einen Stickstoffstrom ersetzt. Nach erfolgter Spülung des Reaktors wurde der Reaktorein- und Ausgang verschlossen und der Reaktor aus der Anlage ausgebaut, so daß der Katalysator ohne Kontakt mit Luft in eine Glove-Box transferiert werden konnte. Bei dem weiteren Katalysatorhandling wie etwa dem Einbau des Katalysators in den Reaktor bzw. in die Analysengeräte wurde ebenfalls ein Kontakt mit Luft vermieden.

Ein XRD (X-Ray diffraction) des Ausbaukatalysators nach der Metathese-Reaktion zeigt die Verbindungen WC und W₂C, daneben Spuren von metallischem Wolfram. WOₓ-Verbindungen sind nicht zu erkennen.

### Beispiel 3: Herstellung eines MoO₃/SiO₂-Katalysators - Kat H

SiO₂ (BASF D11-10, 1.5 mm Stränge) wurde mit einer wäßrigen, verdünnten Lösung von (NH₄)₆Mo₇O₂₄*4H₂O auf Wasseraufnahme getränkt. Danach wurden die Stränge 16 h im Trockenschrank bei 120°C getrocknet. Abschließend wurde der Katalysator am Drehrohrofen bei 593°C in Luft (20 I/h) 1 h lang calciniert und unter trockenem Stickstoff abgekühlt. Der MoO₃-Gehalt betrug 11,1 wt.-%.

### Beispiel 4: Herstellung eines WO₃/SiO₂-Katatysators - Kat J

508,2 g SiO₂ (Shell X970 CY, 3 mm Stränge) wurden bei 500°C (Luft, 50 Uh) vorgetrocknet. Die abgekühlten Stränge wurden in einer Stickstoffatmosphäre mit einer Lösung von 70 g WCl₆ in 1200 ml Ethanol getränkt. Anschließend wurde der Katalysator im Luftstrom (300 Uh, ca. 30 min) getrocknet und 2 h lang bei 550°C in Luft calciniert (50 Uh). Abschließend wurde der Katalysator noch 2 h unter N₂ (50 L/h) bei 850°C getempert. Der WO₃-Gehalt betrug 7,3%.

### A.2 Aktivierung der Katalysator Precursoren gemäß Schritt b) und c)

### Beispiel 5: Aktivierung der Katalysatoren (Methoden 0 - VII)

Die Katalysatoren wurden, außer bei den Vergleichsmethoden 0 und VII, jeweils zuerst mit einem Kohlenwasserstoff in Kontakt gebracht - entweder direkt im Reaktor oder durch Benetzen mit einer Flüssigkeit vor dem Einbau des Katalysators. Die mit Kohlenwasserstoffen angefeuchteten Katalysatoren wurden, außer in den Vergleichsmethoden 0 und VI, anschließend unter fließendem Stickstoff (30 I/h) auf die angegebene Temperatur für ein angegebenes Zeitintervall aufgeheizt und anschließend unter Stickstoff auf die Reaktionstemperatur abgekühlt. Die Bedingungen sind im Einzelnen in Tabelle 2 angegeben.

**Tabelle 2**

| Methode | Kohlenwasserstoff | T (Benetzung) [°C] | Einwirkzeit | T (Tempern) ¹⁾ [°C] | Temper-Dauer [h] |
|---|---|---|---|---|---|
| 0 (Vgl.) | Entfällt | Entfällt | Entfällt | Entfällt | Entfällt |
| I (Vgl.) | 1-Buten | 190 ¹⁾ | 3 h | 300 | 18 |
| II (Vgl.) | 1-Buten | 190¹⁾ | 3 h | 400 | 17 |
| III | 1-Buten | 190 ¹⁾ | 3 - 5 h | 510 | 16-20 |
| IV | n-Octen-1 | Raumtemp. | 10 min | 510 | 15 |
| V ²⁾ | n-Octan | Raumtemp. | 10 min | 510 | 15 |
| VI (Vgl.) | n-Octen-1 | Raumtemp. | 10 min | Entfällt | Entfällt |
| VII (Vgl.) | Entfällt | Entfällt | Entfällt | 510 | 17 |

| | | | | | |
|---|---|---|---|---|---|
| ¹⁾ Angegeben ist die Temperatur am Eingang der Schüttung. Durch eine ungleichmäßige Temperaturverteilung der Heizung nimmt die Temperatur zum Ende der Schüttung hin zu und lag um ca. 10 - 15% höher. ²⁾ Nicht Teil der Erfindung | | | | | |

### In-situ XRD-Messung an Katalysatoren F/0 und F/IV

### Messung an Katalysator F/0

Katalysator F/0 stellt einen Katalysator des Standes der Technik dar und wurde wie unter Punkt A.1, Beispiel 1 beschrieben hergestellt (Methode 0 gemäß Tablle 2). Der Katalysator F/0 wurde pulverisiert. Die Probe wurde auf einem beheizten Korundteller (Tiefe 0.8 mm) in die XRD-Messkammer eingebracht. Bei dem Messgerät handelt es sich um ein Modell D8 Advance (Fa. Bruker/AXS), ausgerüstet mit einer Heizkamera HTK 1200 der Fa. Paar. Gemessen wurde mit Cu-K_{α}-Strahlung in θ/θ-Geometrie mit primär- und sekundärseitigem Göbelspiegel im Bereich 2θ = 15° - 52° mit 6s/step bei ausgewählten Temperaturen.

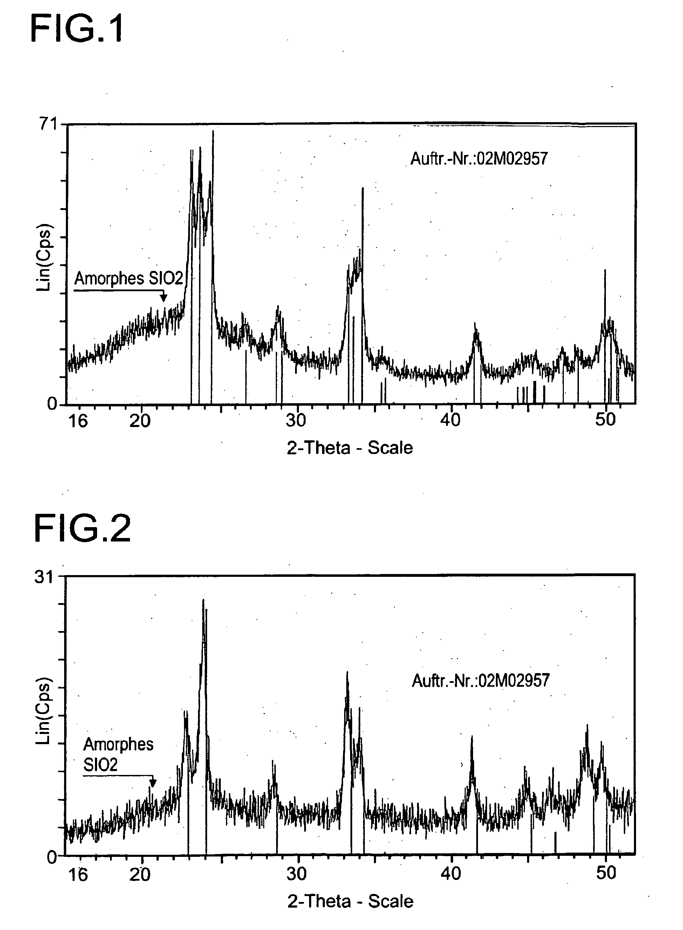

Bei Raumtemperatur lag bei der Probe jeweils monoklines WO₃ und amorphes SiO₂ vor (Bild 1). Die Probe wurde unter Luft aufgeheizt. Bei 400, 500 und 600°C war keine Veränderung festzustellen. Erst bei 850°C bildete sich tetragonales WO₃. Danach wurde auf 200°C abgekühlt, wobei sich das WO₃ wieder in die monokline Phase umlagerte.

### Messung an Katalysator F/IV

Der Katalysator F/0 wurde pulverisiert und mit 1-Octen benetzt. Die Probe wurde nun in die oben beschriebene Messapperatur eingebracht und unter Stickstoffatmosphäre aufgeheizt. Bereits bei 400°C bildete sich tetragonales WO₃ g (Bild 2). Diese Phase blieb bei 500 und 600°C stabil. Im Gegensatz zu Katalysator F/0 erfolgte auch nach Abkühlen auf 200°C keine Umlagerung in die monokline Phase. Hieraus ist abzuleiten, dass Katalysator F/0 durch das erfindungsgemäße Verfahren irreversibel verändert wurde.

### B. Methathesereaktionen

### Beispiele 6 - 25:

Ca. 35 g Katalysator wurden in einen elektrisch beheizten Rohrreaktor gefüllt. Am Eingang des Katalysatorbetts wurde die genannte Temperatur eingestellt. Eine ungleichmäßige Temperaturverteilung der Heizung führte zu einem Anstieg der Temperatur hin zum Ende des Katalysatorbetts (jeweils in Klammern angegeben). Als Feed wurde reines 1-Buten zugefahren. Der Reaktionsdruck war 9,7 bar. Die Analyse des Reaktoraustrags erfolgte on-line mit einem GC: Vor der eigentlichen Messung waren die Katalysatoren nach der jeweils angegebenen Prozedur 0 - VI aktiviert worden. Die Ergebnisse sind in Tabelle 3 angegeben.

**Tabelle 3**

| Bsp. | Kat / Meth. | T [°C] | WHSV h⁻¹ | 1-Buten-Ums. [%] | Isomerisierung¹⁾ [%] | Hexene [mol%] | Propen [mol%] | C₆-Selektivität [mol%]²⁾ |
|---|---|---|---|---|---|---|---|---|
| 5 Vgl.) | B / 0 | 190 (256) | 7,9 | 66,6 | 66,4 | 0 | 0 | 0 |
| 6 (Vgl.) | C2 / 0 | 190 (249) | 7,9 | 24,7 | 24,5 | 0 | 0 | 0 |
| 7 (Vgl.) | D/0 | 190 (268) | 7,6 | 43,6 | 43,4 | 0 | 0,1 | 0 |
| 8 (Vgl.) | E1 / 0 | 190 (263) | 8,0 | 60,3 | 59,9 | 0,1 | 0,2 | 0,2 |
| 9 (Vgl.) | G/0 | 190 (303) | 8,7 | 52,6 | 51,5 | 0,2 | 0,8 | 0,8 |
| 10 (Vgl.) | H/0 | 190 (269) | 7,8 | 9,9 | 79,6 | 0,1 | 0,3 | 0,1 |
| 11 (Vgl.) | J/0 | 189 (242) | 15,1 | 14,0 | 13,0 | 0,3 | 0,2 | 7,9 |
| 12 (Vgl) | A/VII | 189 (274) | 8,0 | 65,7 | 49,3 | 6,3 | 7,7 | 19,3 |
| 13 (Vgl.) | C1 / VI | 190 (247) | 9,3 | 53,4 | 53,4 | 0 | 0 | 0 |
| 14 (Vgl.) | B/I | 190 (280) | 8,1 | 77,4 | 76,0 | 0,4 | 1,4 | 0,9 |
| 15 (Vgl.) | B/II | 190 (270) | 8,1 | 75,0 | 60,1 | 5,0 | 6,9 | 13,3 |
| 16 | B/III | 190 (256) | 8,1 | 62,4 | 43,1 | 7,8 | 9,6 | 24,9 |
| 17 | A/III | 189 (274) | 8,0 | 63,0 | 32,7 | 11,5 | 16,1 | 36,4 |
| 18a | C2/III | 189 (208) | 4,0 | 37,0 | 6,9 | 15,6 | 2,8 | 84,1 |
| 18b | C2/III | 190 (231) | 6,0 | 32,5 | 5,7 | 12,8 | 2,1 | 78,5 |
| 18c | C2/III | 190 (240) | 8,0 | 29,9 | 4,3 | 13,1 | 1,5 | 87,9 |
| 19 | D/III | 200 (244) | 7,6 | 47,8 | 11,5 | 16,8 | 7,8 | 70,3 |
| 20 | E1/III | 190 (251) | 7,4 | 46,5 | 13,5 | 14,6 | 7,3 | 62,8 |
| 21 (Vgl.) | E2/0 | 190 (228) | 7,7 | 43,5 | 23,0 | 8,7 | 7,4 | 40,0 |
| 22 | E2/III | 190 (228) | 7,2 | 47,0 | 12,8 | 16,6 | 6,5 | 70,5 |
| 23a | G/III | 155 (238) | 4,3 | 44,1 | 13,9 | 13,5 | 6,9 | 61,1 |
| 23b | G/III | 190 (293) | 8,8 | 52,8 | 22,5 | 13,4 | 10,2 | 50,8 |
| 24 | H/III | 190 (263) | 7,6 | 64,0 | 57,0 | 2,4 | 3,4 | 7,6 |
| 25a | J/III | 189 (212) | 4,0 | 41,2 | 8,2 | 17,9 | 4,3 | 86,0 |
| 25b | J/III | 190 (237) | 10,0 | 33,9 | 6,1 | 15,1 | 2,8 | 86,7 |
| 25c | J/III | 190 (240) | 14,9 | 38,7 | 4,8 | 16,7 | 2,6 | 86,3 |
| 25d | J/III | 170 (217) | 14,9 | 26,9 | 2,5 | 13,3 | 1,1 | 98,9 |
| 26 | E1/IV | 190 (247) | 7,7 | 43,2 | 14,8 | 12,2 | 6,9 | 56,7 |
| 27 | B/V ³⁾ | 190 (250) | 7,7 | 64,5 | 38,4 | 10,1 | 13,5 | 31,2 |

| | | | | | | | | |
|---|---|---|---|---|---|---|---|---|
| ¹⁾ Summe der gebildeten Allylfragmente = [Propen]/2 + 2-Butene + Pentene ²⁾ C₆-Selektivität = 2*C₆-Ausbeute / Umsatz(1-Buten) ³⁾ Nicht Teil der Erfindung | | | | | | | | |

## Patentansprüche

1. Verfahren zur Herstellung von Trägerkatalysatoren (Katalysator K), umfassend einen Träger (Träger T) und eine Aktivkomponente (Aktivator A), wobei man
a) einen Katalysator-Precursor herstellt, indem man auf einen Träger ausgewählt aus der Gruppe bestehend aus Al₂O₃, Alumosilicate, Ga₂O₃, SiO₂, GeO₂, TiO₂, ZrO₂, SnO₂ und Mischungen der vorgenannten Verbindungen, einen für die Katalyse von Metathesereaktionen üblichen Aktivator (Aktivator-Precursor) der Verbindungen umfasst, die Elemente aus der V., VI. und VII. Nebengruppe enthalten, aufbringt (Schritt a)
b) den gemäß Schritt (a) hergestellten Katalysator-Precursor mit einem C₃- bis C₁₂-Olefin bei einer Temperatur zwischen -20 und 550°C in Kontakt bringt (Schritt b) und
c) den gemäß Schritt (b) hergestellten Katalysator-Precursor in einer Inertgasatmosphäre bei einer Temperatur von 410 bis 850°C tempert (Schritt c).

2. Verfahren nach Anspruch 1, wobei die Verbindungen der V., VI. und VII. Nebengruppe ausgewählt sind aus der Gruppe bestehend aus Sulfiden, Oxiden, Nitriden, Carbiden, Oxycarbiden, Carbonylen, organischen Komplexen, Halogeniden, Säuren, Polysäuren, Heteropolysäuren und Salzen der Säuren, Polysäuren und Heteropolysäuren.

3. Verfahren nach einem der vorstehenden Ansprüche, wobei der Aktivator-Precursor Verbindungen umfasst, die ausgewählt sind aus der Gruppe bestehend aus Rhenium- Wolfram- und Molybdänverbindungen.

4. Verfahren nach einem der vorstehenden Ansprüche, wobei der Aktivator-Precursor Verbindungen umfasst, die ausgewählt sind aus der Gruppe bestehend aus Molybdänoxiden, Wolframcarbiden und Wolframoxiden.

5. Verfahren nach einem der vorstehenden Ansprüche, wobei der Aktivator-Precursor Verbindungen umfasst, die ausgewählt sind aus der Gruppe bestehend aus Metallverbindungen der V., VI. und VII. Nebengruppe und einen Promotor, der ausgewählt ist aus der Gruppe bestehend aus Cobalt-, Alkali- und Erdalkalimetallverbindungen.

6. Verfahren nach einem der vorstehenden Ansprüche, wobei man den Schritt (a) ausführt, indem man den Träger (T) mit einer Lösung des Aktivator-Precursors imprägniert und anschließend trocknet und danach ggf. calciniert.

7. Verfahren nach einem der vorstehenden Ansprüche, wobei man in Schritt (c) ein Inertgas, ausgewählt aus der Gruppe bestehend aus Stickstoff, Kohlendioxid und Edelgas oder Mischungen hiervon einsetzt.

## Claims

1. A process for preparing a supported catalyst (catalyst C) comprising a support (support S) and an active component (activator A), wherein
a) a catalyst precursor is prepared by applying an activator customary for the catalysis of metathesis reactions (activator precursor), which comprises compounds containing elements of transition groups V, VI and VII, to a support selected from the group consisting of Al₂O₃, aluminosilicates, Ga₂O₃, SiO₂, GeO₂, TiO₂, ZrO₂, SnO₂ and mixtures of the abovementioned compounds (step a),
b) the catalyst precursor prepared in step (a) is brought into contact with a C₃- to C₁₂-olefin at from -20 to 550°C (step b) and
c) the catalyst precursor prepared in step (b) is heated at from 410 to 850°C in an inert gas atmosphere (step c).

2. The process according to claim 1, wherein the compounds of elements of transition groups V, VI and VII are selected from the group consisting of sulfides, oxides, nitrides, carbides, oxycarbides, carbonyls, organic complexes, halides, acids, polyacids, heteropolyacids and salts of the acids, polyacids and heteropolyacids.

3. The process according to either of the preceding claims, wherein the activator precursor comprises compounds selected from the group consisting of rhenium, tungsten and molybdenum compounds.

4. The process according to any of the preceding claims, wherein the activator precursor comprises compounds selected from the group consisting of molybdenum oxides, tungsten carbides and tungsten oxides.

5. The process according to any of the preceding claims, wherein the activator precursor comprises compounds selected from the group consisting of compounds of metals of transition groups V, VI and VII and a promoter selected from the group consisting of cobalt, alkali metal and alkaline earth metal compounds.

6. The process according to any of the preceding claims, wherein step (a) is carried out by impregnating the support (S) with a solution of the activator precursor and subsequently drying it and, if desired, calcining it.

7. The process according to any of the preceding claims, wherein the inert gas used in step (c) is selected from the group consisting of nitrogen, carbon dioxide and noble gases and mixtures thereof.

## Revendications

1. Procédé de préparation de catalyseurs supportés (catalyseur K), comprenant un support (support T) et un composant actif (activateur A), dans lequel
a) on prépare un précurseur de catalyseur en ce qu'on applique, sur un support choisi dans le groupe constitué par Al₂O₃, les aluminosilicates, Ga₂O₃, SiO₂, GeO₂, TiO₂, ZrO₂, SnO₂ et les mélanges des composés susmentionnés, un activateur usuel pour la catalyse de réactions de métathèse (précurseur d'activateur) qui comprend des composés contenant des éléments du Vème, VIème et VIIème groupe secondaire (étape a)
b) on met en contact le précurseur de catalyseur préparé selon l'étape (a) avec une oléfine en C₃-C₁₂ à une température entre -20 et 550°C (étape b) et
c) on traite thermiquement le précurseur de catalyseur préparé selon l'étape (b) dans une atmosphère de gaz inerte à une température de 410 à 850°C (étape c).

2. Procédé selon la revendication 1, les composés du Vème, VIème et VIIème groupe secondaire étant choisis dans le groupe constitué par les sulfures, les oxydes, les nitrures, les carbures, les oxycarbures, les carbonyles, les complexes organiques, les halogénures, les acides, les polyacides, les hétéropolyacides et les sels des acides, polyacides et hétéropolyacides.

3. Procédé selon l'une quelconque des revendications précédentes, le précurseur d'activateur comprenant des composés choisis dans le groupe constitué par les composés du rhénium, du tungstène et du molybdène.

4. Procédé selon l'une quelconque des revendications précédentes, le précurseur d'activateur comprenant des composés choisis dans le groupe constitué par les oxydes de molybdène, les carbures de tungstène et les oxydes de tungstène.

5. Procédé selon l'une quelconque des revendications précédentes, le précurseur d'activateur comprenant des composés choisis dans le groupe constitué par les composés métalliques du Vème, VIème et VIIème groupe secondaire et un promoteur choisi dans le groupe constitué par les composés de cobalt, de métal alcalin et de métal alcalino-terreux.

6. Procédé selon l'une quelconque des revendications précédentes, l'étape (a) étant réalisée en ce qu'on imprègne le support (T) d'une solution du précurseur d'activateur, puis on sèche et le cas échéant on calcine.

7. Procédé selon l'une quelconque des revendications précédentes, dans lequel on utilise, dans l'étape (c), un gaz inerte, choisi dans le groupe constitué par l'azote, le dioxyde de carbone et un gaz noble ou leurs mélanges.
